(19) **Europäisches Patentamt / European Patent Office / Office européen des brevets**

(11) **EP 2 735 325 B1**

(12) **EUROPEAN PATENT SPECIFICATION**

(45) Date of publication and mention
of the grant of the patent:
**11.05.2016 Bulletin 2016/19**

(51) Int Cl.:
***A61M 1/16*** *(2006.01)*      ***A61M 1/34*** *(2006.01)*

(21) Application number: **13184170.2**

(22) Date of filing: **12.09.2013**

(54) **Extracorporeal blood treatment apparatus**

Vorrichtung zur extrakorporalen Behandlung von Blut

Appareil de traitement sanguin extracorporel

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **21.11.2012 IT MO20120285**

(43) Date of publication of application:
**28.05.2014 Bulletin 2014/22**

(73) Proprietor: **Zanotti, Monica
41124 Modena (IT)**

(72) Inventor: **Zanotti, Monica
41124 Modena (IT)**

(74) Representative: **Villanova, Massimo et al
Luppi Crugnola & Partners S.r.l.
Viale Corassori, 54
41124 Modena (IT)**

(56) References cited:
**WO-A1-2012/127298      US-A- 6 117 099
US-A1- 2011 240 555      US-B1- 6 860 866**

**Description**

**Background of the invention**

The invention relates to an extracorporeal blood treatment apparatus and a method that can be actuated by the extracorporeal blood treatment apparatus for determining at least one parameter.

Specifically, though not exclusively, the invention can be used during a hemodialysis or hemodiafiltration treatment to determine at least one parameter, such as for example the diffusive dialysance of the filter (membrane treatment device) and/or the ionic concentration of the patient blood.

As is known, in a hemodialysis apparatus the diffusive dialysance D for a certain solute in the blood is the quotient between the solute flow, which passes by diffusion from the blood chamber to the fluid chamber through the membrane, and the difference of solute concentration between the blood chamber and the fluid chamber.

Two known dialysance calculation formulas, the first considering the membrane blood side and the second considering the membrane fluid side, are the following:

$$D = QBi \cdot \frac{CBi - CBo}{\alpha \cdot CBi - CDi} \quad \text{and} \quad D = QDi \cdot \frac{CDi - CDo}{\alpha \cdot CDi - CBi},$$

where QBi is the blood flow at the filter inlet, QDi is the dialysate flow at the filter inlet, CBi and CBo are the solute concentrations in the blood at the inlet and, respectively, outlet of the filter, CDi and CDo are the solute concentrations in the dialysate at the inlet and, respectively, outlet of the filter, $\alpha$ is the Donnan factor.

Hemodiafiltration apparatuses are disclosed in the patent publications US 6939471 and US 2010/168925, wherein a treatment parameter (the hemodiafilter dialysance) is determined by modifying the ionic concentration of the treatment fluid. The patent publication US 6860866 discloses an apparatus as in the preamble of the first claim, in which the value of dialysance or clearance is firstly determined - for a certain dialysis fluid flow rate Qd1, a certain blood flow rate Qb1 and a certain ultrafiltration flow rate Qf1 - measuring the concentrations Cdi1 and Cdo1 of the fresh and spent dialysis fluid by conductivity sensors, increasing the sodium concentration of the fresh dialysis fluid, measuring again the concentrations Cdi2 and Cdo2 of the fresh and spent dialysis fluid; the value of dialysance or clearance on varying the dialysis fluid flow rate Qd(t) and/or the blood flow rate Qb(t) and/or the ultrafiltration flow rate Qf(t) is then calculated, with no further measure of the concentration of the spent dialysis fluid.

In all the above prior art examples, there is the drawback of the need of modifying the prescribed value of the treatment fluid chemical composition at least for a certain time period. Furthermore a system suitable for modifying such chemical composition must be provided.

**Summary of the invention**

An aim of the invention is realizing an extracorporeal blood treatment apparatus that is able to determine a parameter during the treatment.

An aim of the invention is providing a method for determining a parameter during a hemodialysis or hemodiafiltration treatment.

An advantage is determining a parameter during the treatment with no need of modifying the ionic concentration of the fresh treatment fluid.

An advantage is determining a patient parameter and/or a treatment parameter and/or a treatment apparatus parameter, such as for example the filter ionic dialysance (e.g. sodium dialysance) and/or the patient blood ionic concentration (e.g. sodium ion concentration).

These aims and advantages and more besides are attained by the apparatus according to one or more of the appended claims.

In one example, an extracorporeal blood treatment apparatus comprises a blood treatment device and means for determining a parameter on the basis of spent treatment fluid electrical conductivity values measured at different values of ultrafiltration flow through a semipermeable membrane of said device.

Further characteristics and advantages of the present invention will better emerge from the detailed description that follows.

**Brief description of the drawings**

The invention can be better understood and implemented with reference to the attached drawings that show some embodiments thereof by way of non-limiting examples.

Figure 1 is a schematic drawing of a first example of extracorporeal blood treatment apparatus (hemodiafiltration apparatus).

Figure 2 is a schematic drawing of a second example of extracorporeal blood treatment apparatus (hemodiafiltration apparatus).

Figure 3 is a schematic drawing of a third example of extracorporeal blood treatment apparatus (hemodiafiltration apparatus).

Figure 4 is a schematic drawing of a fourth example of extracorporeal blood treatment apparatus (hemodiafiltration apparatus).

Figures 5 and 6 are two diagrams of the fluid system of any one of the apparatuses of Figures 1 to 4 during a hemodiafiltration with pre-dilution in two different configurations.

Figure 7 is a diagram of the fluid system of any one of the apparatuses of Figures 1 to 4 during a treatment without ultrafiltration.

Figure 8 is a diagram of the fluid system of any one of the apparatuses of Figures 1 to 4 during a treatment with ultrafiltration.

**Detailed description**

With reference to the above figures, the elements that are analogous have been indicated, where possible, with the same reference number.

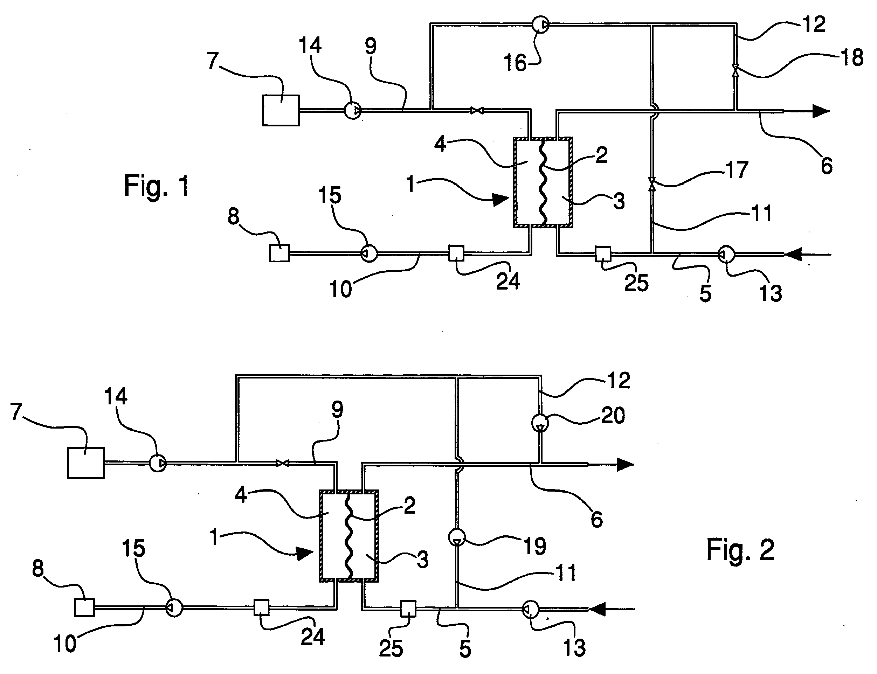

In Figures 1 to 4, 1 denotes a blood treatment device (hemodiafilter), 2 a semipermeable membrane of the device 1, 3 a first chamber of the device 1 (blood chamber belonging to an extracorporeal blood circuit), 4 a second chamber of the device 1 (fluid chamber belonging to a fluid circuit in which a treatment fluid flows, in particular a dialysis fluid), 5 a first line of the extracorporeal blood circuit (blood withdrawal line, or arterial line to withdraw blood from the patient and supply it to the first chamber 3), 6 a second line of the extracorporeal blood circuit (blood return line, or venous line to return blood from the first chamber 3 to the patient), 7 a treatment fluid source, 8 a spent fluid drain, 9 an inlet line of the fluid circuit to supply (fresh) fluid from the source 7 to the second chamber 4, 10 an outlet line to discharge (spent) fluid from the second chamber 4 to the drain 8. 11 denotes a pre-dilution line to supply a substitution fluid to the first line 5 (i.e. to the blood circuit before the device 1), 12 a post-dilution line to supply the substitution fluid to the second line 6 (i.e. to the blood circuit after the device 1).

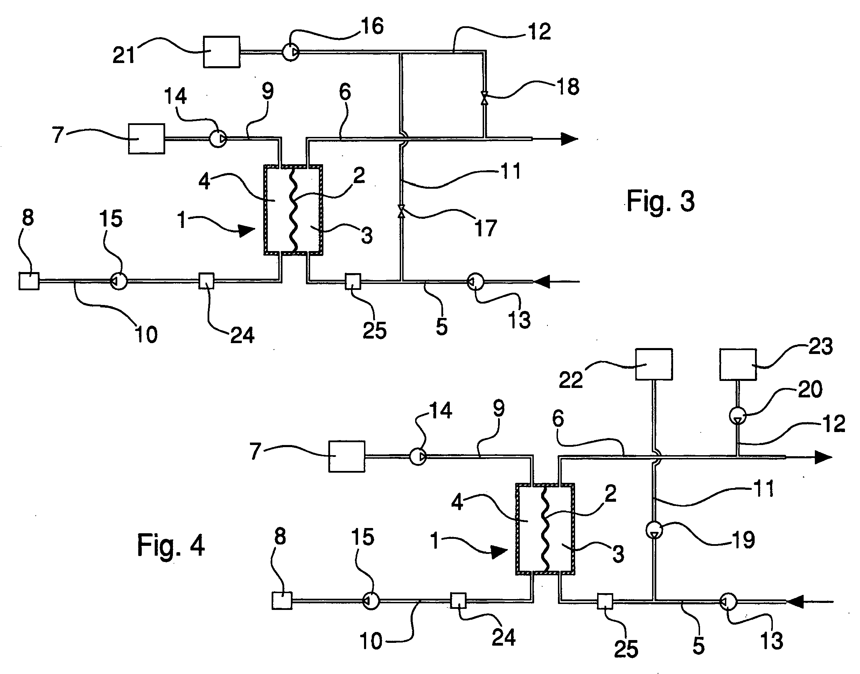

Each apparatus example comprises a system of actuators for controlling the blood flow in the extracorporeal blood circuit and/or the substitution fluid flow (in the pre-dilution line 11 and/or in the post-dilution line 12) and/or the ultrafiltration flow through the semipermeable membrane 2. The system of actuators may comprise, in particular, a blood pump 13 and/or a fresh fluid pump 14 and/or a spent fluid pump 15. The system of actuators may comprise, in particular for the apparatuses of Figures 1 and 3, a substitution fluid pump 16 and/or a pre-dilution valve 17 and/or a post-dilution valve 18. The system of actuators may comprise, in particular for the apparatuses of Figures 2 and 4, a pre-dilution pump 19 and/or a post-dilution pump 20.

The apparatus of Figure 3 comprises, in particular, a substitution fluid source 21 that feeds the pre-dilution line 11 and the post-dilution line 12.

The apparatus of Figure 4 comprises, in particular, a pre-dilution fluid source 22 connected to the pre-dilution line 11 and a post-dilution fluid source 23 connected to the post-dilution line 12.

Each apparatus example may comprise a sensor 24 for detecting a physical-chemical characteristic of the fluid in the outlet line 10. This sensor 24 may comprise, for example, an electrical conductivity sensor.

Each apparatus example may comprise a further sensor 25 for detecting a physical-chemical characteristic (for example the solid component concentration) of the blood in the extracorporeal blood circuit, in particular in the first line 5. This further sensor 25 may comprise, for example, a hematocrit sensor.

Each apparatus example may comprise an ultrafiltration control system comprising a programmable electronic control unit connected to ultrafiltration sensor means that may comprise, for example, flow sensors, not shown, for detecting the inlet flow in the inlet line 9 and the outlet flow in the outlet line 10. These flow measures may be used for controlling the inlet and/or outlet flows to have a desired ultrafiltration flow Qfset.

The ultrafiltration control system may be of any other known type, such as for example of the balance chambers type with ultrafiltration pumping means (for example as that shown by US 6939471).

Each of the fluid sources, indicated by 7, 21, 22, 23, may comprise an on-line fluid preparation system (using water and concentrates), or a batch type source (for example one or more ready-to-use fluid containers).

In the apparatuses of Figures 1 and 2, the pre-dilution line 11 and post-dilution line 12, which are connected (in branching relationship) with the inlet line 9, may be supplied with at least a part of the treatment fluid (dialysis fluid) flowing in the inlet line 9. Each apparatus may be provided with a purification system (for example by ultrafiltration), not shown, to make this treatment fluid suitable as substitution fluid. This purification system may comprise one or more ultrafilters (arranged in series), for example two ultrafilters as in US6939471.

Each hemodiafiltration apparatus herein described may comprise an apparatus designed for chronic kidney disease periodical treatments as well as an apparatus designed for acute renal failure intensive treatments.

Each hemodiafiltration apparatus, which has been schematically described in the various examples, may comprise any further element comprised in any hemodiafiltration apparatus of known type. For example, each extracorporeal blood circuit may comprise one or more other elements (such as for example an arterial expansion chamber and/or a venous expansion chamber and/or a pump segment and/or a patient sensor and/or an air bubble sensor and/or an anticoagulant line and/or at least one auxiliary line and/or a safety venous clamp, etc.) provided in blood lines used in hemodiafiltration apparatuses of known type. Each hydraulic circuit of non-corporal fluids may comprise one or more other elements (such as for example a disinfectant and/or wash system and/or a degassing system and/or an ultrafilter rinsing system, etc.) provided in hydraulic circuits of hemodiafiltration apparatuses of known type.

The hemodiafiltration apparatuses, in the above described examples, may comprise both a pre-dilution line and a post-dilution line. In other examples, hemodiafiltration apparatuses may be provided with no (or with more than one) pre-dilution line and/or with no (or with more than one) post-dilution line. When both the pre-dilution line and the post-dilution line are absent, the hemodiafiltration apparatus is actually a hemodialysis apparatus (with no substitution fluid).

The invention may concern a hemofiltration apparatus, wherein the fresh fluid flow at the inlet of the fluid chamber 4 is null (the inlet line 9 is optional).

Each of the above described hemodiafiltration apparatuses determines at least one parameter (patient parameter and/or treatment parameter and/or apparatus parameter; for example the hemodiafilter ionic dialysance and/or the ionic concentration in the blood, in particular of a certain solute, for example sodium ion) during the treatment from at least two spent fluid physical-chemical parameter values measured (by means of the electrical conductivity sensor 24) at two different ultrafiltration flows though the semipermeable membrane.

The electronic control unit is programmed to actuate a method for determining at least one (patient, treatment or apparatus) parameter during the treatment. The present invention also relates to this parameter determining method. The programmable control unit is provided with program instructions for implementing at least some operations of the method, for example described as follows.

In particular the control unit may be programmed to set a first configuration of the hemodiafiltration apparatus with a first ultrafiltration flow Qf1 through the semipermeable membrane 2. The control unit may be programmed to set a second configuration of the hemodiafiltration apparatus with a second ultrafiltration flow Qf2 different from Qf1. At least one of the two flows Qf1 and Qf2 may be null or negative (wherein a negative flow flows from the second chamber 4, or fluid chamber, to the first chamber 3, or blood chamber).

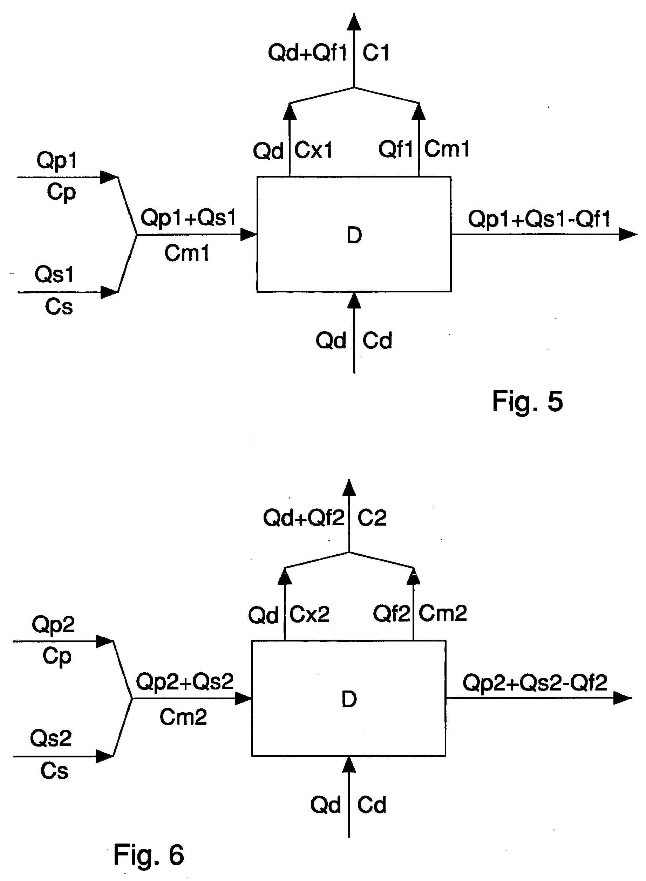

Figures 5 and 6 represent a diagram of a fluid system that comprises the treatment device 1, in the two configurations with flows Qf1 and Qf2, when at least one pre-dilution line is present.

The symbols that have been used, in particular in Figures 5 and 6, denote as follows:

Qp1 = flow of plasma (blood liquid component) in the first line upstream the hemodiafilter in the first operative configuration (Figure 5);

Qp2 = flow of plasma in the first line in the second operative configuration (Figure 6);

Qs1 = substitution fluid flow in the pre-dilution line in the first operative configuration;

Qs2 = substitution fluid flow in the pre-dilution line in the second operative configuration;

Cp = plasma solute concentration;

Cs = substitution fluid solute concentration in the pre-dilution line;

Cm1 = solute concentration in the fluid (mix of blood and possible pre-dilution fluid) at the blood chamber inlet in the first operative configuration;

Cm2 = solute concentration in the fluid (mix of blood and possible pre-dilution fluid) at the blood chamber inlet in the second operative configuration;

Qd = dialysis fluid flow at the fluid chamber inlet (the same in both configurations);

Cd = dialysis fluid solute concentration at the fluid chamber inlet (the same in both configurations);

Qf1 = ultrafiltration flow through the membrane 2 in the first operative configuration;

Qf2 = ultrafiltration flow through the membrane 2 in the second operative configuration;

C1 = spent fluid solute concentration in the first operative configuration (determined by means of the electrical conductivity sensor 24);

C2 = spent fluid solute concentration in the second operative configuration (determined by means of the electrical conductivity sensor 24);

D = diffusive dialysance of the hemodiafilter for the solute.

Cx1 and Cx2 denote two values (not actual, but fictitious values) related to the spent dialysis fluid conductivity, due only to diffusion, in the two different configurations. In other words, it is considered, for sake of simplicity, that the actual spent dialysis fluid conductivity (C1 and C2) is determined partly by the diffusion effect, which determines the conductivity variation from the dialysis fluid conductivity value Cd at the inlet (dialysate side) to the dialysis fluid fictitious conductivity values (Cx1 and Cx2) at the outlet (dialysate side), and partly by the ultrafiltration effect due to the passage through the membrane 2 of a fluid (at least partly blood plasma, if the flow is positive from the blood side to the dialysate side) at certain concentrations (Cml and Cm2) and at certain flows (Qf1 and Qf2).

The mass balance of the system in the two configurations, considering that the diffusive dialysance D of the blood treatment device 1 remains constant at the two different ultrafiltration flows Qf1 and Qf2, leads to the following mathematic system of six equations:

$$(1) \quad (Qp1 + Qs1) * Cm1 = Qp1 * Cp + Qs1 * Cs$$

$$(2) \quad D = Qd * \frac{Cx1 - Cd}{Cm1 - Cd}$$

$$(3) \quad (Qd + Qf1) * C1 = Qd * Cx1 + Qf1 * Cm1$$

$$(4) \quad (Qp2 + Qs2) * Cm2 = Qp2 * Cp + Qs2 * Cs$$

$$(5) \quad D = Qd * \frac{Cx2 - Cd}{Cm2 - Cd}$$

$$(6) \quad (Qd + Qf2) * C2 = Qd * Cx2 + Qf2 * Cm2$$

The equations (2) and (5) of the dialysance D can be considered admissible approximations. The Donnan factor $\alpha$ may be introduced to take into account the Donnan effect.

The known terms of the equation system are Qp1, Qp2, Qs1, Qs2, Cs, Qd, Cd, Qf1,Qf2,C1,C2.

Qp1 and Qp2 may be set by controlling the flow Qb of the blood pump and knowing the hematocrit value measured by the correspondent sensor.

Qs1 and Qs2 are known, for example on the basis of the flows of the pre-dilution pump 19 in the two configurations.

The concentrations Cs, Cd and Qd are, generally, known or controllable values, normally compelled on the basis of the medical prescription.

The ultrafiltration flows Qf1 and Qf2 are also known, e.g. as being determined or set by the ultrafiltration system.

The concentrations C1 and C2 are determined (in a known manner) on the basis of the values measured by the sensor 24.

The unknowns of the equation system are Cp, D, Cm1, Cm2, Cx1, Cx2. The system of six equations and six unknowns may be, generally, solved. It must be considered Cm1 ≠ Cd, Cm2 ≠ Cd. In practice it is possible to simplify the system, for example imposing that Qs2 = 0 and/or Qs1 = 0 and/or Qf1 or Qf2 = 0 and/or Cs = Cd.

In the above-described mathematical model it is assumed that the diffusive dialysance D of the hemodiafilter does not change in the two configurations. In case of pre-dilution, this assumption may be more effective if, for example, the flow of the liquid component at the first chamber inlet is the same in the two configurations, i.e. when Qp1 + Qs1 = Qp2 + Qs2. To this aim the blood flow and the pre-dilution flow may be appropriately controlled so as to obtain Qp1 + Qs1 = Qp2 + Qs2, for example using the hematocrit sensor to determine (in a known manner) the plasma flows Qp1 and Qp2 from the blood flows Qb1 and Qb2.

The method for determining the parameter during the hemodiafiltration treatment (method at least partly implemented by computer program instructions) may comprise the steps of: providing a blood treatment device having a first chamber and a second chamber separated by a semipermeable membrane; supplying blood to said first chamber through a first line of an extracorporeal blood circuit; withdrawing blood from said first chamber through a second line of said extracorporeal blood circuit; (optionally) supplying fresh fluid to said second chamber through an inlet line of a fluid circuit; withdrawing spent fluid from said second chamber through an outlet line of said fluid circuit; (optionally) supplying a substitution fluid to said blood circuit through at least one dilution line; controlling an ultrafiltration flow through said semipermeable membrane; detecting two values of a physical-chemical characteristic of the spent fluid in said outlet line at two different ultrafiltration flows; and determining a patient parameter and/or a treatment parameter and/or an apparatus parameter from said two detected values.

The parameter may be determined on the basis of a mathematical model of the mass balance of a system that includes the blood treatment device, in particular considering that the diffusive dialysance of the blood treatment device remains the same at said two different ultrafiltration flows.

Example

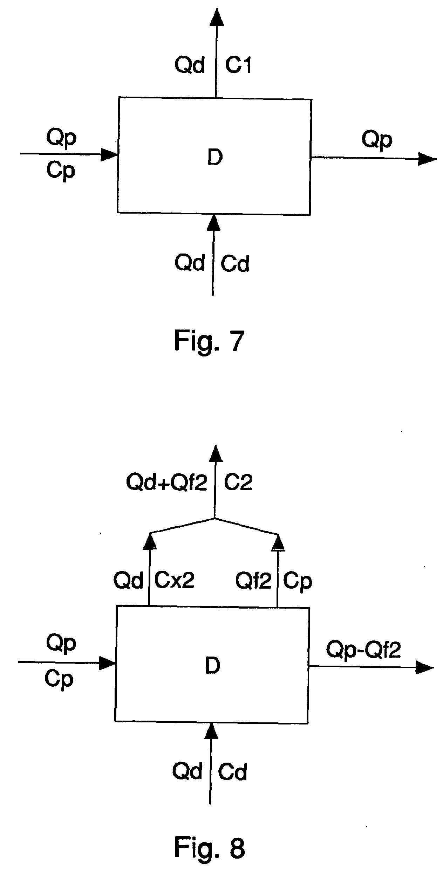

The first configuration of this example is that represented in Figure 7 (hemodialysis configuration) in which $Qs1 = 0$ and $Qf1 = 0$.

In this case (absence of pre-dilution and ultrafiltration) it will be $Cx1 = C1$ and $Cm1 = Cp$. Replacing these terms into equations (1), (2) and (3) the following equation will be obtained;

$$(7) \quad D = Qd * \frac{C1 - Cd}{Cp - Cd}.$$

The second configuration is that represented in Figure 8 (hemodiafiltration with post-dilution configuration) in which $Qs2 = 0$ and $Qf2 \neq 0$. In this case (ultrafiltration with no pre-dilution) it will be $Cm2 = Cp$ and $Qp2 = Qp1 = Qp$. Replacing into equations (4), (5) and (6), two equations are obtained as follows:

$$(8) \quad D = Qd * \frac{Cx2 - Cd}{Cp - Cd};$$

$$(9) \quad (Qd + Qf2) * C2 = Qd * Cx2 + Qf2 * Cp.$$

The solution of the system of three equations (7), (8) and (9) with three unknowns D, Cx2 and Cp is as follows:

$$Cx2 = C1$$

$$Cp = C2 + \frac{Qd}{Qf2} * (C2 - C1)$$

$$D = Qd * \frac{Qf2(C1 - Cd)}{Qf2(C2 - Cd) + Qd(C2 - C1)}$$

It is thus possible to calculate both the blood plasma ionic concentration Cp and the dialysance D of the blood treatment membrane device.

The parameter (membrane device dialysance D and/or blood ionic concentration) can be determined, from at least two spent treatment fluid conductivity values measured at at least two different ultrafiltration flow values, by other mathematical models and/or by empirical type methods. For example, in another mathematical model (based on the mass balance of the system in the two different ultrafiltration configurations), the dialysance D (when ultrafiltration is not null) can be calculated in a (known) manner different from that above described.

**Claims**

1. Apparatus comprising:

    - a blood treatment device (1) having a first chamber (3) and a second chamber (4) separated by a semipermeable membrane (2);
- an extracorporeal blood circuit having a first line (5) to supply blood to said first chamber (3) and a second line (6) to withdraw blood from said first chamber (3);
- a fluid circuit having an inlet line (9) to supply fresh fluid to said second chamber (4) and an outlet line (10) to withdraw spent fluid from said second chamber (4);
- ultrafiltration means for actuating an ultrafiltration flow through said semipermeable membrane (2);
- sensor means (24) for detecting **a characteristic** of the spent fluid in said outlet line (10);

**characterized by** comprising means for determining at least one parameter from two values of said **characteristic** of the spent fluid measured at two different ultrafiltration flows (Qf1; Qf2)**, said** at **least one parameter comprising the dialysance (D) of the blood treatment device (1) and/or a chemical-physical parameter of the blood.**

2. Apparatus according to claim 1, comprising at least one dilution line (11; 12) to supply a substitution fluid to said blood circuit.

3. Apparatus according to claim 2, wherein said two values are detected at two different substitution fluid flows in said dilution line.

4. Apparatus according to claim 2 or 3, wherein said at least one dilution line comprises a pre-dilution line (11) to supply a substitution fluid to said first line (5).

5. Apparatus according to claim 4, wherein said two values are measured at two different substitution fluid flows in said pre-dilution line (11).

6. Apparatus according to any preceding claim, wherein said means for determining comprises a programmable electronic control unit provided with computer program instructions, in particular for determining said parameter during an hemodialysis or hemodiafiltration treatment.

7. Apparatus according to any preceding claim, wherein said means for determining determines said at least one parameter on the basis of a mathematical model of the mass balance in a system comprising the blood treatment device (1), in particular taking into account that the dialysance (D) of the blood treatment device (1) remains the same at said two different ultrafiltration flows.

8. Apparatus according to any preceding claim, wherein said means for determining determines said at least one parameter from a mathematical model based on the following equations:

$$(Qp1 + Qs1) * Cm1 = Qp1 * Cp + Qs1 * Cs$$

$$D = Qd * \frac{Cx1 - Cd}{Cm1 - Cd}$$

$$(Qd + Qf1) * C1 = Qd * Cx1 + Qf1 * Cm1$$

$$(Qp2 + Qs2) * Cm2 = Qp2 * Cp + Qs2 * Cs$$

$$D = Qd * \frac{Cx2 - Cd}{Cm2 - Cd}$$

$$(Qd + Qf2) * C2 = Qd * Cx2 + Qf2 * Cm2$$

wherein:

Qf1 = first ultrafiltration flow;
Qf2 = second ultrafiltration flow ≠ Qf1;
Qp1 = plasma flow at the first ultrafiltration flow;
Qp2 = plasma flow at the second ultrafiltration flow;
Qs1 = substitution fluid flow in a pre-dilution line at the first ultrafiltration flow;
Qs2 = substitution fluid flow in the pre-dilution line at the second ultrafiltration flow;
Cp = plasma solute concentration;
Cs = substitution fluid solute concentration;
Cm1 = solute concentration of the fluid at the blood chamber inlet at the first ultrafiltration flow;
Cm2 = solute concentration of the fluid at the blood chamber inlet at the second ultrafiltration flow;
Qd = fresh fluid flow at the fluid chamber inlet;
Cd = fresh fluid solute concentration;
C1 = spent fluid solute concentration at the first ultrafiltration flow;
C2 = spent fluid solute concentration at the second ultrafiltration flow;
D = dialysance of the blood treatment device;
Cx1 = fictitious spent fluid solute concentration due to diffusion at the first ultrafiltration flow;
Cx2 = fictitious spent fluid solute concentration due to diffusion at the second ultrafiltration flow.

9. Apparatus according to any preceding claim, wherein a first (Qf1) of said two different ultrafiltration flows (Qf1; Qf2) is null.

10. Apparatus according to claim 9, wherein said at least one parameter comprises the blood plasma ionic concentration Cp determined with the formula

$$Cp = C2 + \frac{Qd}{Qf2} * (C2 - C1),$$

and/or the dialysance D of the blood treatment device (1) determined with the formula

$$D = Qd * \frac{Qf2(C1 - Cd)}{Qf2(C2 - Cd) + Qd(C2 - C1)},$$

wherein:

Qd = flow of the fresh fluid in the inlet line, kept at the same value at said two different ultrafiltration flows (Qf1, Qf2);
Qf2 = second, non-null, ultrafiltration flow;
Cd = ionic concentration of the fresh fluid;
C1 = ionic concentration of the spent fluid measured at the first, null, ultrafiltration flow Qf1;
C2 = ionic concentration of the spent fluid measured at the second ultrafiltration flow Qf2.

11. Apparatus according to any preceding claim, **wherein said characteristic comprises an electrical property of the spent fluid.**

12. Apparatus **according to claim 11, wherein said electrical property comprises the electrical conductivity of the spent fluid.**

13. Apparatus according to **any preceding** claim , wherein said chemical-physical parameter of the blood comprises the ionic concentration (Cp) of the blood.

**Patentansprüche**

1. Gerät umfasst:

- eine Blutbehandlungsvorrichtung (1) mit einer ersten Kammer (3) und einer zweiten Kammer (4) durch eine semipermeable Membran (2) getrennt;

- einen extrakorporalen Blutkreislauf mit einer ersten Leitung (5) zum Zuführen von Blut zur ersten Kammer (3) und einer zweiten Leitung (6) zur Entnahme von Blut aus der ersten Kammer (3);
- einen Fluidkreislauf mit einer Eingangsleitung (9) zur Zuführung von Frischflüssigkeit in die zweite Kammer (4) und einer Auslassleitung (10) zum Abziehen der verbrauchten Flüssigkeit aus der zweiten Kammer (4);
- Ultrafiltrationmittel, um eine Flussrate der Ultrafiltration durch die semipermeable Membran (2) durchzuführen;
- Sensormittel (24) zum Messen einer Eigenschaft der verbrauchten Flüssigkeit in der Ausgangsleitung (10);

**dadurch gekennzeichnet, dass** sie Mittel zur Bestimmung mindestens eines Parameters aus den beiden Werten der Eigenschaft der verbrauchten Flüssigkeit ab, gemessen bei zwei verschiedenen Fließraten von Ultrafiltration ($Qf1$, $Qf2$); wobei zumindest ein Parameters umfasst die Dialysance D der Blutbehandlungsvorrichtung (1) und/oder einen chemisch-physischen Parameter des Blutes.

2. Gerät nach Anspruch 1, mit mindestens einer Dilutionleitung (11; 12) zum Zuführen einer Substitutionsflüssigkeit zu den Blutkreislauf.

3. Gerät nach Anspruch 2, wobei die beiden Werte auf zwei unterschiedlichen Fließraten von Substitutionsflüssigkeit in der Dilutionleitung gemessen werden.

4. Gerät nach Anspruch 2 oder 3, wobei mindestens eine Dilutionleitung eine Prädilutionleitung (11) zum Zuführen einer Substitutionsflüssigkeit an die erste Leitung (5) umfasst.

5. Gerät nach Anspruch 4, wobei die beiden Werte auf zwei unterschiedlichen Fließraten der Substitutionsflüssigkeit in der Prädilutionleitung (11) gemessen werden.

6. Gerät nach einem der vorhergehenden Ansprüche, wobei die Bestimmungsmittel eine mit Computerprogrammanweisungen versehene programmierbare elektronische Steuerungseinheit umfassen insbesondere für die Bestimmung des Parameters während einer Behandlung von Hämodialyse oder Hämodiafiltration.

7. Gerät nach einem der vorhergehenden Ansprüche, wobei die Bestimmungsmittel zumindest einen Parameter bestimmen, basierend auf einem mathematischen Modell der Massenbilanz in einem System, das die Blutbehandlungsvorrichtung (1) umfasst, insbesondere während die Dialysance (D) die Blutbehandlungsvorrichtung (1) in den zwei verschiedenen Fließraten von Ultrafiltration unveränder bleibt.

8. Gerät nach einem der vorhergehenden Ansprüche, wobei die Bestimmungsmittel zumindest einen Parameter bestimmen, aus einem mathematischen Modell auf der Grundlage der folgenden Gleichungen:

$$(Qp1 + Qs1) * Cm1 = Qp1 * Cp + Qs1 * Cs$$

$$D = Qd * \frac{Cx1 - Cd}{Cm1 - Cd}$$

$$(Qd + Qf1) * C1 = Qd * Cx1 + Qf1 * Cm1$$

$$(Qp2 + Qs2) * Cm2 = Qp2 * Cp + Qs2 * Cs$$

$$D = Qd * \frac{Cx2 - Cd}{Cm2 - Cd}$$

$$(Qd + Qf2) * C2 = Qd * Cx2 + Qf2 * Cm2$$

wobei:

Qf1 = erste Ultrafiltrationsflussrate;

Qf2 = zweite Ultrafiltrationsflussrate ≠ Qf1;

Qp1 = Plasmafluss um die erste Ultrafiltrationsflussrate;

Qp2 = Plasmafluss um die zweite Ultrafiltrationsflussrate;

Qs1 = Flussrate der Substitutionsflüssigkeit in einer Prädilutionleitung um die erste Ultrafiltrationsflussrate;

Qs2 = Flussrate der Substitutionsflüssigkeit in der Prädilutionleitung um die zweite Ultrafiltrationsflussrate;

Cp = Solutekonzentration in dem Plasma;

Cs = Solutekonzentration in der Substitutionsflüssigkeit;

Cm1 = Solutekonzentration in der Flüssigkeit am Eingang der Blutkammer, um die erste Ultrafiltrationsflussrate;

Cm2 = Solutekonzentration in der Flüssigkeit am Eingang der Blutkammer um die zweite Ultrafiltrationsflussrate;

Qd = Flussrate der frischen Flüssigkeit am Eingang der Fluidkammer;

Cd = Solutekonzentration in der frischen Flüssigkeit;

C1 = Solutekonzentration in der verbrauchten Flüssigkeit um die erste Ultrafiltrationsflussrate;

C2 = Solutekonzentration in der verbrauchten Flüssigkeit um die zweite Ultrafiltrationsflussrate;

D = Dialysance der Blutbehandlungsvorrichtung;

Cx1 = fiktive Solutekonzentration in der verbrauchten Flüssigkeit aufgrund der Diffusion um die erste Ultrafiltrationsflussrate;

Cx2 = fiktive Solutekonzentration in der verbrauchten Flüssigkeit aufgrund der Diffusion um die zweite Ultrafiltrationsflussrate.

9. Gerät nach einem der vorhergehenden Ansprüche, bei dem die erste (Qf1) der zwei verschiedenen Fließraten von Ultrafiltration (Qf1, Qf2) null ist.

10. Gerät nach Anspruch 9, bei dem zumindest ein Parameter durch die folgende Formel bestimmte Ionenkonzentration des Blutplasmas Cp umfasst

$$Cp = C2 + \frac{Qd}{Qf2} * (C2 - C1),$$

und/oder die Dialysance D der Blutbehandlungsvorrichtung (1) nach der Formel

$$D = Qd * \frac{Qf2(C1 - Cd)}{Qf2(C2 - Cd) + Qd(C2 - C1)},$$

wobei:

Qd = Flussrate der frischen Flüssigkeit in der Eingangsleitung, auf dem gleichen Wert mit den beiden verschiedenen Fließraten von Ultrafiltration (Qf1, Qf2) in Übereinstimmung gehalten;

Qf2 = zweite Ultrafiltrationsflussrate, ungleich null;

Cd = Ionenkonzentration in der frischen Flüssigkeit;

C1 = Ionenkonzentration in der verbrauchten Flüssigkeit gemessen an der ersten Ultrafiltrationsflussrate Qf1, die null ist;

C2 = Ionenkonzentration in der verbrauchten Flüssigkeit gemessen an der zweiten Ultrafiltrationsflussrate Qf2.

11. Gerät nach einem der vorhergehenden Ansprüche, wobei die Eigenschaft eine elektrische Eigenschaft der verbrauchten Flüssigkeit umfasst.

12. Gerät nach Anspruch 11, wobei die elektrische Eigenschaft die elektrische Leitfähigkeit der verbrauchten Flüssigkeit umfasst.

13. Gerät nach einem der vorhergehenden Ansprüche, wobei der chemisch-physischer Parameter des Blutes die Ionenkonzentration (Cp) des Blutes umfasst.

**Revendications**

1. Appareil comprenant:

   - un dispositif de traitement du sang (1) ayant une première chambre (3) et une seconde chambre (4) séparées par une membrane semi-perméable (2);
   - un circuit extracorporel de sang ayant une première ligne (5) pour fournir du sang à ladite première chambre (3) et une deuxième ligne (6) pour prélever du sang de ladite première chambre (3);
   - un circuit de fluide ayant une ligne d'entrée (9) pour fournir du fluide frais à ladite seconde chambre (4) et une ligne de sortie (10) pour prélever du fluide usé de ladite seconde chambre (4);
   - des moyens de ultrafiltration pour actionner une débit d'ultrafiltration à travers ladite membrane semi-perméable (2);
   - des moyens de détection (24) pour détecter une caractéristique du fluide usé dans ladite ligne de sortie (10);

   **caractérisé en ce qu'**il comprend des moyens pour déterminer au moins un paramètre à partir de deux valeurs de ladite caractéristique du fluide usé mesurée à deux débits d'ultrafiltration différents (Qf1 Qf2); ledit au moins un paramètre comprenant la dialysance (D) du dispositif de traitement du sang (1) et/ou un paramètre physico-chimique du sang.

2. Appareil selon la revendication 1, comprenant au moins une ligne de dilution (11; 12) pour fournir un fluide de substitution à ledit circuit de sang.

3. Appareil selon la revendication 2, dans lequel lesdites deux valeurs sont détectées à deux débits différents de fluide de substitution dans ladite ligne de dilution.

4. Appareil selon la revendication 2 ou 3, dans lequel ladite au moins une ligne de dilution comporte une ligne de pré-dilution (11) pour fournir un fluide de substitution à ladite première ligne (5).

5. Appareil selon la revendication 4, dans lequel lesdites deux valeurs sont mesurées à deux débits différents de fluide de substitution dans ladite ligne de pré-dilution (11).

6. Appareil selon l'une quelconque des revendications précédentes, dans lequel ledit moyen pour déterminer comprend une unité de commande électronique programmable pourvue d'instructions de programme d'ordinateur, en particulier pour la détermination dudit paramètre pendant un traitement d'hémodialyse ou hémodiafiltration.

7. Appareil selon l'une quelconque des revendications précédentes, dans lequel ledit moyen pour déterminer détermine ledit au moins un paramètre sur la base d'un modèle mathématique de l'équilibre de masse dans un système comprenant le dispositif de traitement du sang (1), en particulier en tenant compte du fait que la dialysance (D) du dispositif de traitement du sang (1) reste à la même dans lesdits deux débit d'ultrafiltration différents.

8. Appareil selon l'une quelconque des revendications précédentes, dans lequel ledit moyen pour déterminer détermine ledit au moins un paramètre à partir d'un modèle mathématique basé sur les équations suivantes:

$$(Qp1 + Qs1) * Cm1 = Qp1 * Cp + Qs1 * Cs$$

$$D = Qd * \frac{Cx1 - Cd}{Cm1 - Cd}$$

$$(Qd + Qf1) * C1 = Qd * Cx1 + Qf1 * Cm1$$

$$(Qp2 + Qs2) * Cm2 = Qp2 * Cp + Qs2 * Cs$$

$$D = Qd * \frac{Cx2 - Cd}{Cm2 - Cd}$$

$$(Qd + Qf2) * C2 = Qd * Cx2 + Qf2 * Cm2$$

dans lesquels:

Qf1 = premier débit d'ultrafiltration;
Qf2 = second débit d'ultrafiltration $\neq$ Qf1;
Qp1 = débit de plasma au premier débit d'ultrafiltration;
Qp2 = débit de plasma au deuxième débit d'ultrafiltration;
Qs1 = débit de fluide de substitution dans une ligne de pré-dilution au premier débit d'ultrafiltration;
Qs2 = débit de fluide de substitution dans la ligne de pré-dilution au deuxième débit d'ultrafiltration;
Cp = concentration du soluté dans le plasma;
Cs = concentration du soluté dans le fluide de substitution;
Cm1 = concentration du soluté dans le fluide à l'entrée de la chambre de sang au premier débit d'ultrafiltration;
Cm2 = concentration du soluté dans le fluide à l'entrée de la chambre de sang au deuxième débit d'ultrafiltration;
Qd = débit de fluide frais à l'entrée de la chambre de fluide;
Cd = concentration du soluté dans le fluide frais;
C1 = concentration du soluté dans le fluide usé au premier débit d'ultrafiltration;
C2 = concentration du soluté dans le fluide usé au deuxième débit d'ultrafiltration;
D = dialysance du dispositif de traitement du sang;
Cx1 = concentration fictive du soluté dans le fluide usé due à la diffusion au premier débit d'ultrafiltration;
Cx2 = concentration fictive du soluté dans le fluide usé due à la diffusion au deuxième débit d'ultrafiltration.

9. Appareil selon l'une quelconque des revendications précédentes, dans lequel un premier (Qf1) desdits deux différents débits d'ultrafiltration (Qf1, Qf2) est nulle.

10. Appareil selon la revendication 9, dans lequel ledit au moins un paramètre comprend la concentration ionique Cp du plasma sanguin déterminé avec la formule

$$Cp = C2 + \frac{Qd}{Qf2} * (C2 - C1),$$

et/ou la dialysance D du dispositif de traitement du sang (1) déterminé avec la formule

$$D = Qd * \frac{Qf2(C1 - Cd)}{Qf2(C2 - Cd) + Qd(C2 - C1)},$$

dans lesquels:

Qd = débit du fluide frais dans la ligne d'entrée, maintenue à la même valeur à ces deux débits d'ultrafiltration différents (Qf1, Qf2);
Qf2 = seconde, non nul, débit d'ultrafiltration;
Cd = concentration ionique du fluide frais;
C1 = concentration ionique du fluide usé mesurée à la première, nul, débit d'ultrafiltration Qf1;
C2 = concentration ionique du fluide usé mesurée à la deuxième débit d'ultrafiltration Qf2.

11. Appareil selon l'une quelconque des revendications précédentes, dans lequel ladite caractéristique comprend une propriété électrique du liquide usé.

12. Appareil selon la revendication 11, dans lequel ladite propriété électrique comprend la conductivité électrique du fluide usé.

**13.** Appareil selon l'une quelconque des revendications précédentes, dans lequel ledit paramètre physico-chimique du sang comprend la concentration ionique (Cp) du sang.

Fig. 1

Fig. 2

Fig. 3

Fig. 4

EP 2 735 325 B1

Fig. 5

Fig. 6

Fig. 7

Fig. 8

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6939471 B
- US 2010168925 A

- US 6860866 B